# EUROPEAN PATENT APPLICATION

(11) **EP 4 846 936 A1**
(43) Date of publication of application: **02.12.2026**
(21) Application number: 26181274.7
(22) Date of filing: 27.05.2026
(51) Int. Cl.: A61B 5/11, A61B 5/00, G16H 50/20, G16H 50/30

(54) **AI BASED MOBILITY DETECTION**

(30) Priority: 27.05.2025 US 202563812457 P
(71) Applicant: Hill-Rom Services, Inc., Batesville, IN 47006 (US)
(72) Inventor: BHAI, Aziz Ali, Batesville, 47006 (US)
(74) Representative: Reddie & Grose LLP

(57) **Abstract**

A patient support apparatus comprises a plurality of sensors, and a control system. The control system includes a controller operable to receive a separate signal from each of the sensors and process the signals to determine at least one patient motion and calculate an assessment factor associated with the patient on the patient support apparatus. The assessment factor may provide an indication of the likelihood of patient injury and/or the extent of patient recovery. The assessment factor may provide a probability associated with the likelihood of patient injury and/or a probability associated with the likelihood and/or speed of patient recovery.

## Description

The present disclosure relates to the use of sensors on a patient support apparatus, such as a hospital bed, to detect patient movement and characterize patient motion based on the movement of different parts of a patient body. More specifically, the present disclosure is directed to an AI motion classifier which assesses movement of different parts of a patient body based on the amplitude and duration to predict recovery and risk of injury.

The use of load cells in patient support apparatuses, such as hospital beds, for example, to measure patient weight is known. Over time, approaches to using the information from the load cells to detect patient movement and to issue an alert or notification when the patient moves beyond a particular threshold have been developed. The use of load cells to make these determinations and inferences based on the motion or movement is limited by the potential for external influences, such as the addition of equipment to the frame supported on the scale. When this is done, the existing information regarding the position of the patient is compromised as the weight distribution is changed unexpectedly.

The pressure sensors used to measure air pressure in zones of an inflatable mattress are used to control the inflation pressure in the zones to control the interface pressure experienced by a patient supported on the mattress. Periodic patient repositioning is crucial to minimize extended exposure of a given part of a patient's body to elevated pressure. This reduction of exposure reduces risk of pressure injury. This repositioning could occur by the patient himself, or it could be assisted by a caregiver. If the movement is patient induced, then it could also be an indicator for the health and recovery of the patient.

It is important to determine patient motion relative to the patient support apparatus and caregiver aid. Movement in this context means a change in position of the patient on the patient support apparatus, such as rolling over or moving toward an edge of a patient support apparatus to exit the patient support apparatus. Movement of different parts of a patient's body may contribute to such motion and a patient's motion may be indicative of a patient's health and/or medical needs. Thus, there is a need to improve the approaches to measuring and characterizing a patient's motion based on the movement of different parts of a patient's body.

The present disclosure includes one or more of the following features alone or in any combination.

According to a first aspect of the present disclosure, a patient support apparatus comprises a patient support apparatus, including a plurality of load cells positioned on the patient support apparatus, a plurality of sensors configured to measure movement of one or more body parts of a patient, and a control system including a controller operable to receive a separate signal from each of the plurality of load cells positioned on the patient support apparatus and each of the plurality of sensors, the control system further including a processor and a memory device, the memory device including a set of instructions that, when executed by the processor, causes the control system to determine at least one patient motion and calculate an assessment factor associated with the patient on the patient support apparatus.

According to a second aspect of the present disclosure, a method of assessing patient recovery, comprises receiving a separate signal from one or more sensors by a control system operable to receive a plurality of signals, processing the signal to determine movement of one or more body parts of a patient by a processor in the control system based on a set of instructions stored in a memory device of the control system, providing an output about at least one patient motion from the processor, and calculating an assessment factor associated with the patient.

According to a third aspect of the present disclosure, a patient support apparatus, comprises a plurality of sensors configured to measure movement of one or more body parts of a patient on the patient support apparatus, and a control system including a controller operable to receive a separate signal from each of the plurality of sensors, the control system further including a processor and a memory device, the memory device including a set of instructions that, when executed by the processor, causes the control system to determine at least one patient motion associated with the patient on the patient support apparatus.

According to a fourth aspect of the present disclosure, a patient support apparatus, comprises a base frame, a plurality of casters supporting the base frame, an upper frame adjustable relative to the base frame, a plurality of movable siderails positioned near the sides of the upper frame, a plurality of load cells positioned on the patient support apparatus and configured to sense a patient weight, a plurality of sensors configured to measure movement of one or more body parts of a patient, a user interface configured to control the patient support apparatus and including a display, and a control system including a controller operable to receive a separate signal from each of the plurality of load cells positioned on the patient support apparatus and each of the plurality of sensors, the control system further including a processor and a memory device, the memory device including a set of instructions, wherein the instructions are generated by artificial intelligence or utilize Artificial Intelligence (AI), wherein the instructions, when executed by the processor, cause the control system to determine at least one patient motion and calculate a score associated with the risk of the patient on the patient support apparatus developing a pressure ulcer.

The invention will now be further described by way of example with reference to the accompanying drawings, in which:
Fig. 1 is a perspective view of a patient support apparatus including a plurality of sensors according to the present disclosure;
Fig. 2 is a block diagram of a portion of a control system configured to measure and process signals from the sensors associated with the patient support apparatus of Fig. 1;
Fig. 3 is a diagrammatic illustration of the interaction between a first frame of the patient support apparatus of Fig. 1 and a second frame supported on load cells supported from the first frame;
Fig. 4 is a side view of a portion of the patient support apparatus of Fig. 1 showing a first frame supported on load cells supported on a second frame, the load cells supporting all of the load of the first frame;
Fig. 5 illustrates a generalized algorithm for processing sensor data and determining a mobility score from one or more sensors on the patient support apparatus;
Fig. 6 illustrates a rule-based algorithm implemented to determine a patient mobility score of a patient on the patient support apparatus;
Fig. 7 is a graphical representation illustrating the type of motion a patient has engaged in the hours leading up to an evaluation or current time; and
FIG. 8 is a graphical representation illustrating patient motion as a function of time.

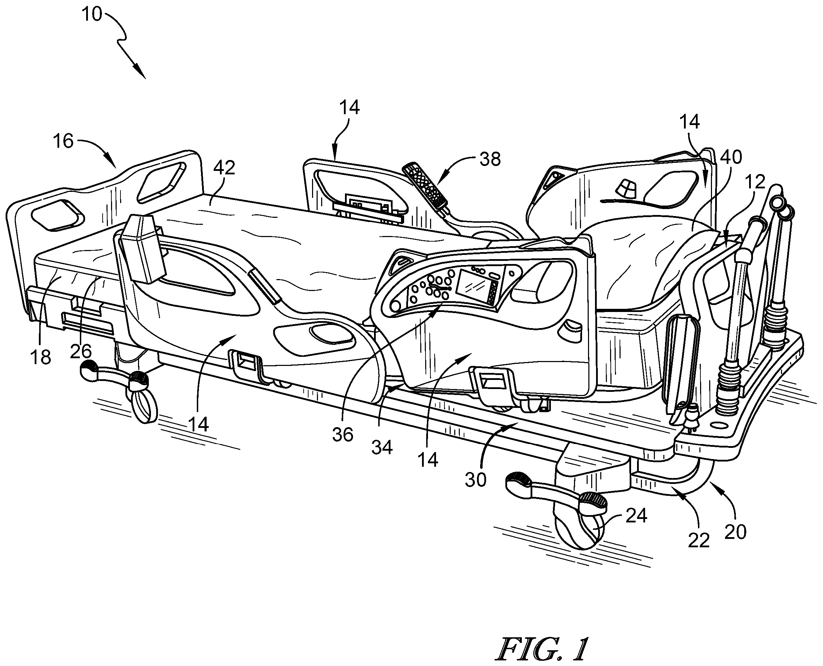

An illustrative patient support apparatus 10 embodied as a hospital bed is shown in FIG. 1. The bed 10 of FIG. 1 has a frame 20 which includes a base frame 22 supported on casters 24. The stationary base frame 22 further supports a weigh frame 30 and an adjustably positionable mattress support upper frame 34 supporting a mattress 18. The mattress 18 is an inflatable patient support surface which includes a plurality of inflatable zones 26 or air bladders 26. The bed 10 further includes a headboard 12 at a head end 40 of the bed 10, a footboard 16 at a foot end 42 of the bed 10, and movable siderails 14 coupled to the upper frame 34 of the bed 10. The bed 10 may also include a user interface 36 positioned on one of the siderails 14. The bed 10 of the embodiment of FIG. 1 is conventionally configured to adjustably position the upper frame 34 relative to the base frame 22 to adjust the position of a patient supported on the mattress 18.

Conventional structures and devices may be provided to adjustably position the upper frame 34, and such conventional structures and devices may include, for example, linkages, drives, and other movement members and devices coupled between base frame 22 and the weigh frame 30, and/or between weigh frame 30 and upper frame 34. Control of the position of the upper frame 34 and mattress 18 relative to the base frame 22 or weigh frame 30 is controlled, for example, by a patient control pendant 38 or user interface 36. The upper frame 34 may, for example, be adjustably positioned in a general incline from the head end 40 to the foot end 42 or vice versa. Additionally, the upper frame 34 may be adjustably positioned such that the head section of the mattress 18 is positioned between minimum and maximum incline angles, e.g., 0-65 degrees, relative to horizontal or bed flat, and the upper frame 34 may also be adjustably positioned such that a seat section (not shown) of the mattress 18 is positioned between minimum and maximum bend angles, e.g., 0-35 degrees, relative to horizontal or bed flat. Those skilled in the art will recognize that the upper frame 34 or portions thereof may be adjustably positioned in other orientations, and such other orientations are contemplated by this disclosure.

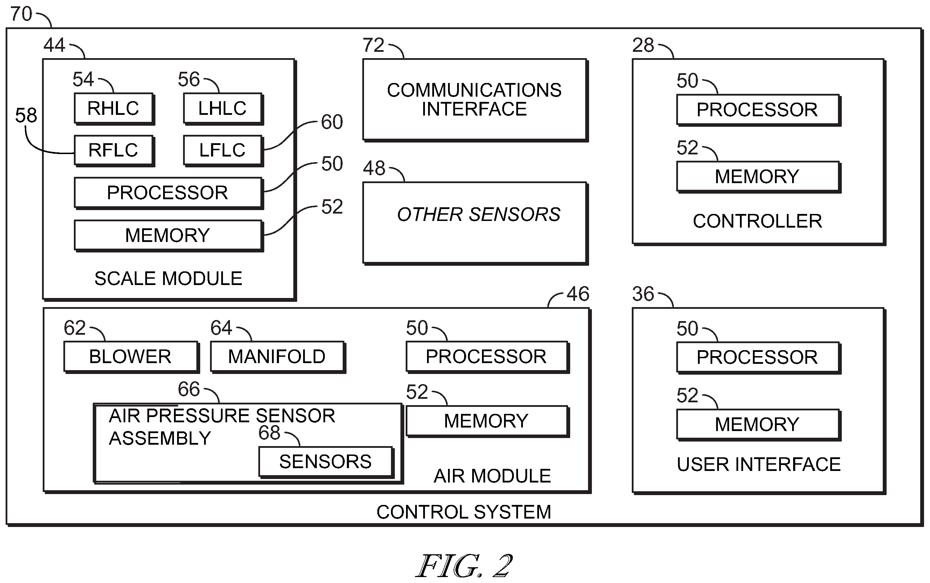

In one illustrative embodiment shown diagrammatically in FIG. 2, the bed 10 has a control system 70 that includes a controller 28, a scale module 44, an air module 46, other sensors 48 (e.g., video sensors, RFID, temperature sensors etc.), and the user interface 36. In the illustrative embodiment each of the controller 28, scale module 44, air module 46, and user interface 36 includes a processor 50 and a memory device 52. The memory device 52 includes instructions that, when executed by the processor 50, cause the processor 50 to perform functions as associated with the particular one of controller 28, scale module 44, air module 46, and user interface 36. The components of the control system 70 communicate amongst themselves to share information and distribute the functions of the bed 10. The processor 50 of each of the controller 28, scale module 44, air module 46, and user interface 36 is also operable, based on instructions from the memory device 52, to communicate with the others of the controller 28, scale module 44, air module 46, and user interface 36 using a communications protocol. It should be understood that the term processor here includes any microprocessor, microcontroller, processor circuitry, control circuitry, preprogrammed device, or any structure capable of accessing the memory device and executing non-transient instructions to perform the tasks, algorithms, and processes disclosed herein. In the illustrative embodiment, the control system 70 employs a conventional controller area network (CAN) 72 for communications between subsystems, but it should be understood that any of a number of networking and communications solutions could be employed in the control system 70.

The scale module 44 includes four load cells 54, 56, 58, and 60. To determine a weight of a patient supported on the mattress 18, the load cells 54, 56, 58, 60 are positioned between the weigh frame 30 and the upper frame 34. Each load cell 54, 56, 58, 60 is configured to produce a signal indicative of a load supported by the respective load cell 54, 56, 58, 60 from the upper frame 34 relative to the weigh frame 30. Some of the structural components of the bed 10 will be designated hereinafter as "right", "left", "head" and "foot" from the reference point of an individual lying on the individual's back on the mattress 18 with the individual's head oriented toward the head end 40 of the bed 10 and the individual's feet oriented toward the foot end 42 of the bed 10. Following this convention, the load cell 54 is designated as the right head load cell (RHLC) in the figures to represent that the load cell positioned at the right side of the bed 10 at the head end 40. The load cell 56 is designated as the left head load cell (LHLC), the load cell 58 is designated as the right foot load cell (RFLC), and the load cell 60 is designated left foot load cell (LFLC), each following the same convention.

The scale module 44 includes a processor 50 that is in communication with each of the respective load cells 54, 56, 58, 60 and operable to process the signals from the load cells 54, 56, 58, 60. The memory device 52 is also utilized by the controller 28 to store information corresponding to features and functions provided by the bed 10. The load cells 54, 56, 58, 60 provide information that is used to establish the actual weight supported on the bed 10 as disclosed in U.S. Patent No. 10,054,479 titled "BED WITH AUTOMATIC WEIGHT OFFSET DETECTION AND MODIFICATION", which is incorporated herein for the disclosure of monitoring and updating a patient load to establish a static patient weight.

In the present disclosure, the independent signals from each of the load cells 54, 56, 58, 60 are used to draw inferences about the patient motion. The independent signals from each of the load cells 54, 56, 58, 60 are especially used to draw inferences about assisted and unassisted patient motion.

The air module 46 is the functional controller for the mattress 18 and includes processor 50 and a memory device 52. The processor 50 is in communication with a blower 62, a manifold 64, and an air pressure sensor assembly 66. The air module 46 is a conventional structure with the manifold 64 operating under the control of the processor 50 to control the flow of air from the blower 62 into and out of a plurality of zones 26 to control the interface pressure experienced by the patient supported on the mattress 18. The air pressure sensor assembly 66 includes separate pressure sensors 68 for measuring the air pressure in each of zones 26. The air pressure sensor assembly 66 includes at least one pressure sensor 68 in each of the zones 26.

The signals from the pressure sensors 68 are useful in making inferences regarding patient movement associated with different body parts and, when used synergistically with the information gleaned from the signals from the load cells 54, 56, 58, 60 and other sensors 48, provide a more fulsome and accurate analysis of patient motion and/or any motion associated with the patient support apparatus 10.

Several types of patient motions including but not limited to rolling to the right, rolling to the left, sitting up, reaching, and/or turning over may be all self-induced (e.g., unassisted) or assisted motions. Some of these motions such as turning over or sitting up for extended duration are classified as transition motions and others such as a quick sit up/lay down or reaching to get something from a side table are classified as non-transition motions. The movement of different body parts of a patient is determined by the pressure sensors 68, the load cells 54, 56, 58, 60, and/or other sensors 48. The movement of different body parts is categorized based on amplitude and duration of movements. Instructions stored in the memory device 52 are configured to process the data from the pressure sensors 68 and/or other sensors 48 to determine patient motion as function of the measured movements of different body parts. For example, different combinations of body part movements having different amplitude and duration may indicate different patient motion such as rolling to the right, rolling to the left, sitting up, reaching, and/or turning over. The occurrence of granular movements involving mostly only upper part or only the lower part of the patient body or involving one side versus the other can also be used to indicate the portion of the body that is being compromised due to lack of movements. For example, if the patient mainly moves lower part of the body, then patient motion would be classified to assume that the upper part of the body may not be moving. Signals from the pressure sensors 68, the load cells 54, 56, 58, 60 and other sensors 48 will be used to determine if the patient motion is assisted, unassisted, a transition motion, and/or a non-transition motion based on the determination of the movement of different body parts.

Once the type of patient motion has been determined, the control system 70 is configured to process the data based on instructions stored in the memory device 52 to determine a probability or score associated with injury risk and patient recovery. For example, if the patient is exposed to an elevated pressure without extended motion, the patient may have an elevated risk associated with risk of injury due to pressure ulcers.

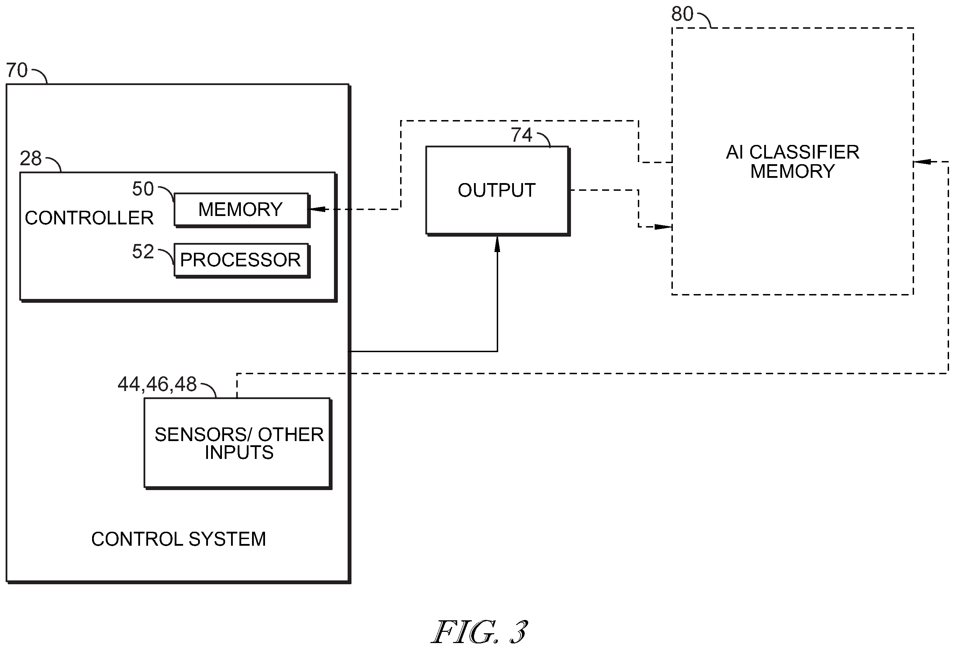

Additionally, as shown in the FIG. 3, data from the pressure sensors 68, the load cells 54, 56, 58, 60, and/or other sensors 48 that is used to determine patient motion as a function of the measured movements of different body parts is provided as an output 74 to an Al classifier 80. The Al classifier 80 is configured to improve upon and automatically update the instructions stored in the memory device 52 that are used to identify future patient motion. Such real time updating of the instructions stored in the memory device 52 helps in the development of personalized motion detection criteria for each patient and in the development of motion detection for specific indications.

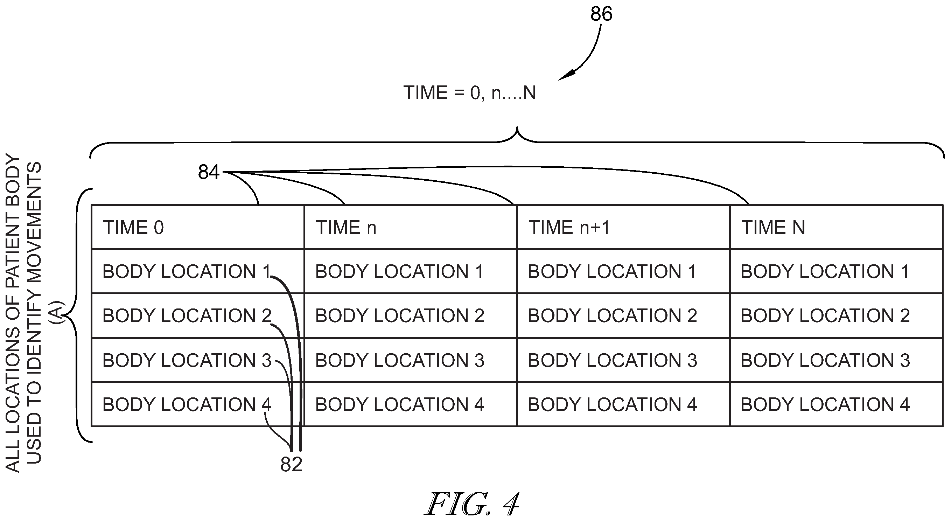

In one embodiment, as shown in FIG. 4, an array of length A is used to store information associated with each body part 82 being monitored, where A is the number of body parts 82 being monitored. Each entry in the array is updated at predetermined frequency 84 (e.g., every minute) over a pre-determined period of time 86 to indicate movement or no movement of that body part 82. The movement may be further classified as assisted or unassisted movement. Further, patient motion including but not limited to rolling to the right, rolling to the left, sitting up, reaching, and turning is determined based on the movement of one or more body parts 82 as evaluated over the pre-determined period of time 86 (e.g., 15 minutes or 30 minutes). In some embodiments, the patient motion may be a compound motion including multiple discrete motions, and each of the discrete motions may be distinguished by the movement of one more body parts 82. The patient motion may be classified as assisted, unassisted, transition, or non-transition motion. In some embodiments, a circular buffer implementation may be used to store data associated with different body parts 82.

Periodically, at predetermined intervals, the entire circular buffer will be evaluated and an average value associated with movement of each body part 82 being monitored and patient motion in general will be determined. In some embodiments, average value associated with movement of each body part 82 may depend on the confidence level used for such determination. This predetermined interval may be the pre-determined period of time 86. These average values may be graphically displayed for caregiver readout over some past duration of time. Such duration of time could be selected by the caregiver or could be pre-configured based on the bed or facility preference. Additionally, or alternatively, the averages may also be presented such as icons with three different colors (green, yellow or orange) and shapes (stop, warning etc.). Additionally, or alternatively, these values may be transmitted for remote processing such as to calculate a Braden score or a discrete mobility score considering other relevant inputs for a given patient. In some embodiments, the Braden score may be sent to a remote computer system or to a patient electronic medical record system.

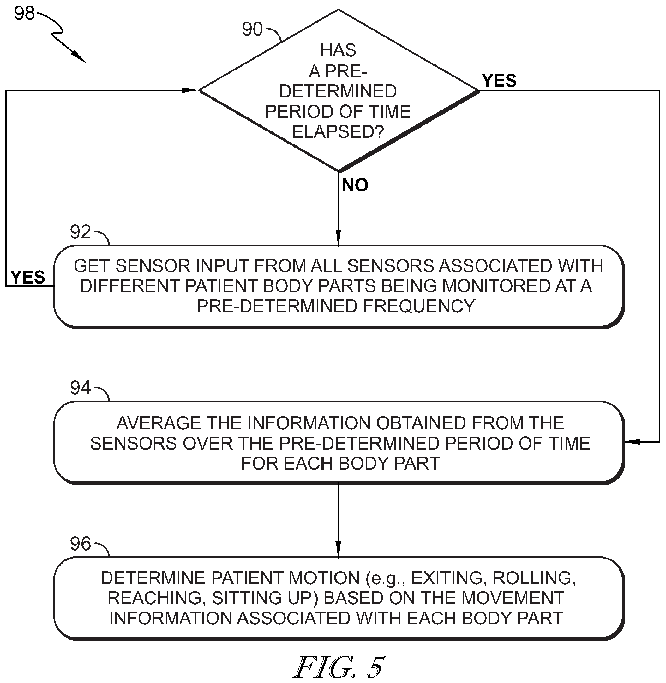

In one embodiment, as shown in FIG. 5, a method 98 of determining patient motion comprises several steps. The control system 70 is configured to determine if the pre-determined period of time 86 has elapsed in step 90. If the predetermined interval has not elapsed, the control system continues to get information obtained from all the pressure sensors 68, the load cells 54, 56, 58, 60 and other sensors 48 at the predetermined frequency 84 in step 92. If the pre-determined period of time 86 has elapsed, the control system averages the information obtained from all the pressure sensors 68, the load cells 54, 56, 58, 60 and other sensors 48 and determines movement information associated with each of the body parts 82 in step 94. This information may include but is not limited to amplitude, duration, frequency etc. Based on the information, and the instructions stored in the memory device 52 and updated from the Al classifier 80, the control system 70 is configured to determine and provide an assessment factor or score associated with patient motion in step 96.

The extent and nature of patient motion can indicate skin exposure to pressure points as well as motion indicating recovery. For example, rolling from one side to the other can minimize pressure injury. Similarly, momentary unassisted sitting up in bed or reaching out to the side table may be a good sign of recovery. In some embodiments, the assessment factor may be in the forms of a patient injury risk factor and/or a patient recovery factor. The assessment factor may provide an indication of the likelihood of patient injury and/or the extent of patient recovery. The assessment factor may provide a probability associated with the likelihood of patient injury and/or a probability associated with the likelihood and/or speed of patient recovery.

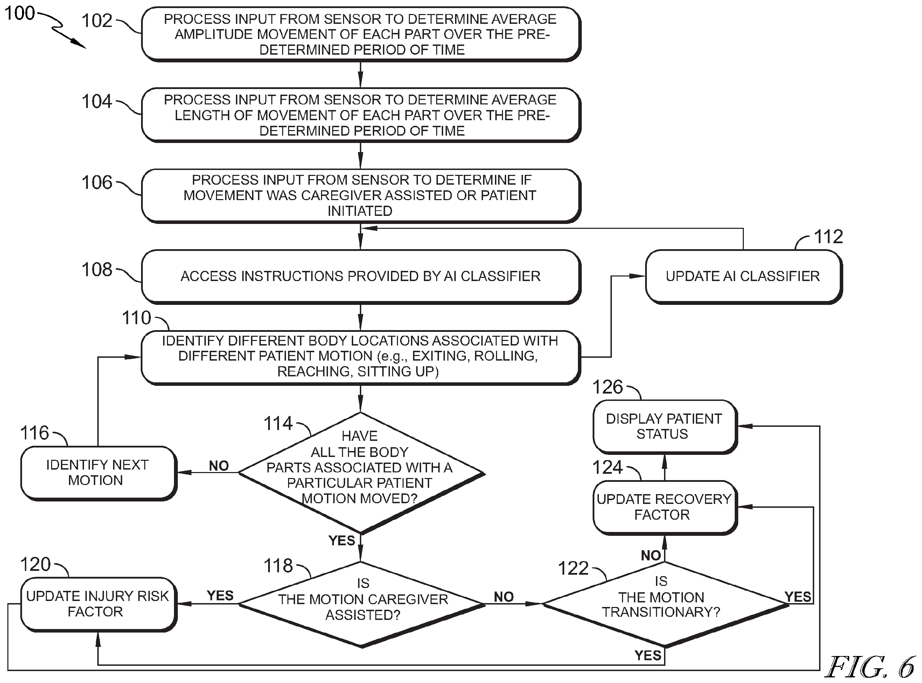

In one embodiment, as shown in FIG. 6, a method 100 of determining an assessment factor associated with a patient comprises several steps. The control system 70 is configured to determine if an injury risk factor and/or a recovery factor associated with a patient has changed over time. The control system 70 is configured to process input signals from sensors 48 to determine average amplitude movement of each body part of the patient over the pre-determined period of time 86 in step 102. The control system 70 is configured to process input signals from sensors 48 to determine average length of movement of each body part 82 of the patient over the pre-determined period of time 86 in step 104. The control system 70 is configured to process input signals from sensors 48 to determine if patient motion was unassisted or caregiver assisted in step 106. The control system 70 is configured to access instructions provided by the AI classifier 80 in step 108. This information may be stored in a memory device 52. The control system 70 is configured to identify and evaluate different body parts associated with each of the different motions including but not limited to exiting, rolling, reaching and sitting up in step 110. The control system 70 is configured to determine movement associated with the different body parts and update the Al classifier 80 in step 112.

In some embodiments, the Al classifier 80 may be updated in real-time or in periodic intervals. In some embodiments, the Al classifier 80 may be updated to determine personalized information for different patients or for different indications. If the control system 70 determines that all the body parts associated with a certain motion have not moved in step 114, the control system 70 executes step 110 for a different motion. If the control system 70 determines that all the body parts associated with a certain motion have moved in step 114, the control system 70 determines if the motion is caregiver assisted in step 118. If the control system 70 determines that the motion is caregiver assisted in step 118, the control system 70 updates the assessment factor associated with injury risk for that patient in step 120. If the control system 70 determines that the motion is not caregiver assisted in step 118, the control system 70 determines if the motion is transitionary in step 122. If the control system 70 determines that the motion is transitionary in step 122, the control system 70 updates the assessment factor associated with injury risk for that patient in step 120 and updates the assessment factor associated with recovery for that patient in step 124. If the control system 70 determines that the motion is not transitionary in step 122, the control system 70 updates the assessment factor associated with recovery for that patient in step 124. The recovery factor and the risk injury factor are output on a display in step 126. In some embodiments, the processor 50 of the control system 70 is configured to process signals from the pressure sensors 68, the load cells 54, 56, 58, 60 and other sensors 48 to execute the method 100 illustrated in FIG. 6.

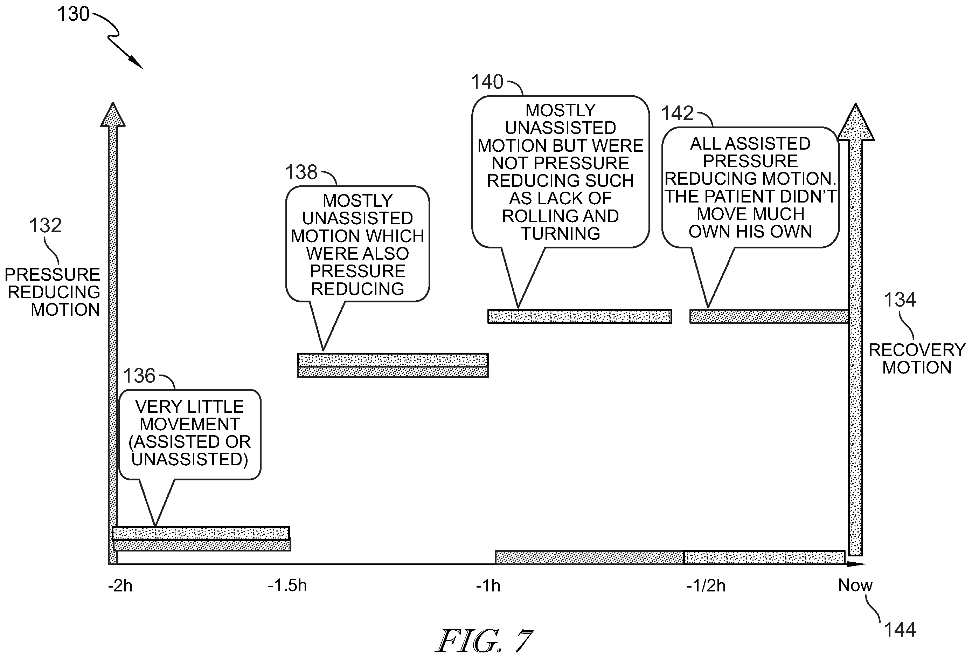

In some embodiments, the Al classifier 80 may be implemented to keep track of motion types and predict and/or notify a caregiver of pressure injury risk or lack of recovery motion associated with a patient. As shown in FIG. 7, in some embodiments, the display may show a graph 130 indicating the type of motion the patient has engaged in the hours leading up to an evaluation or current time 144. In one example, the graph 130 illustrates a plot of pressure reducing motion 132 and recovery motion 134 as a function of time. A low value for a recovery motion 134 may indicate a higher risk of deterioration or a lower rate of recovery. A low value of pressure reducing motion 132 indicates a higher risk of pressure injury. In this example, the patient has engaged in very little assisted or unassisted motion 136 2 hours to 1.5 hours prior to the current time 144. The patient has engaged in mostly unassisted pressure reducing motion 138 about 1.5 hours to 1 hour prior to the current time 144. The patient has engaged in mostly unassisted but not pressure reducing motion 140 about 1 hour to 0.5 hour prior to the current time 144. The patient has engaged in all assisted pressure reducing motion 142 from about 0.5 hours prior to the current time 144.

A lack of motion by the patient could result in the patient experiencing excessive friction and shear force. For example, a patient lacking ability to self-position himself would have higher chance of drifting towards the foot end 42 and then would need assistance to be pulled up in bed 10. A higher head of bed angle (HOB) and/or a lower knee angle could also accelerate drifting towards the foot end 42. In some embodiments, the output 74 of the patient motion assessment from the above implementation may comprise motion trend data reflecting different types of motion. In some embodiments, the output 74 of the patient motion assessment from the above implementation can be further combined with bed positions, using the AI classifier or a non-Al method, related bed function use (such as pull up in bed or indications of pull up in bed such as lowered HOB and assisted shift of the weight towards HOB) and other relevant inputs to calculate friction and shear force the patient has been experiencing over some period of time.

In some embodiments, the risk of pressure injury may be used to automatically adjust the bed 10 to mitigate the risk to patient health. In other embodiments, an alert may be provided to a caregiver based on the risk of pressure injury. The alert may be a visual or audible alert.

In some other embodiments, the assessment factor provided by the control system 70 may be a mobility score such as a Braden score. The Braden score may be used to assess the risk associated with a patient developing a pressure ulcer. In some embodiments, the user interface 36 may be used to collect parameters to determine a Braden score associated with a patient based on information being processed by the AI classifier 80. In some embodiments, the Braden score may be used to adjust the pressure in the plurality of inflatable zones 26 or air bladders 26. In some embodiments, the output 74 of the patient motion assessment from the above implementation may additionally, or alternatively comprise mobility, friction, and/or shear values associated with a patient based on information being processed by the Al classifier 80. The output 74 may be a numerical indicator of the patient state. The numerical indicator may range from 1 to 4 or from 1 to 3. The display may indicate current output values and the historical output value in the form of a time graph.

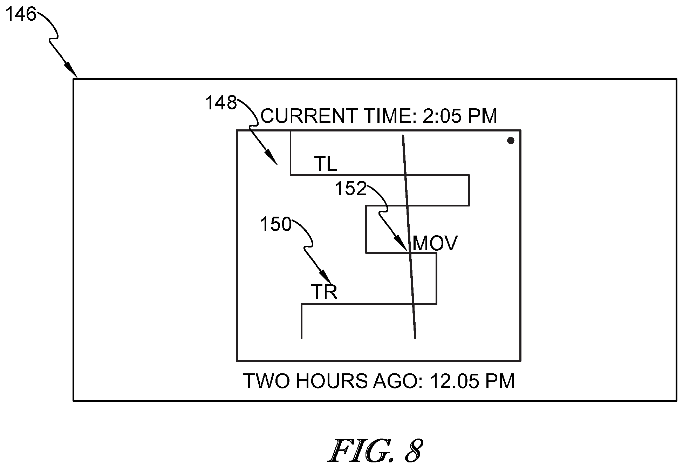

In some embodiments, as shown in FIG. 8, the output 74 may be a graphical representation 146 of patient motion as a function of time over a given time period (e.g., past two hours). The graphical representation 146 shows lateral position of the patient on the patient support apparatus 10. In some embodiments, the graphical representation 146 may also include horizontal position of the patient on the patient support apparatus 10. In some embodiments, the graphical representation 146 may also include Al generated activity symbols identifying motion transition.

In FIG. 8, TL 148 indicates that the patient is a left turned position, TR 150 indicates that the patient is in a right turned position, and MOV 152 indicates that the patient has moved. MOV 152 indicates patient movement that may include patient turning. In some embodiments, patient hyperactivity, such as patient restlessness may also be indicated on the graphical representation 146. In some embodiments, patient attempt to exit the patient support apparatus 10 may also be indicated on the graphical representation 146. In some embodiments, the occurrence of any patient exit alarm may also be indicated on the graphical representation 146. Such an output 74 can allow caregivers to assess likelihood of the patient exiting and potentially falling. In some embodiments, the graphical representation 146 or some other component of the patient support apparatus 10 may display recommendations to the caregiver such as directions to turn the patient left or right. In some embodiments, the graphical representation 146 may include icons and/or legends to better user interaction.

In some embodiments, the graphical representation 146 may be used to assess patient mobility and recovery. In some embodiments, the graphical representation 146 may illustrate overall patient movement over a given time period (e.g., every 2 hours, every 1 hours etc.) as quantified mobility score. In some embodiments, the mobility score may include patient movement over 24 hours. Such a mobility score may be indicative of patient recovery and/or mobility trend. In some embodiments, the graphical representation 146 may illustrate patient movement strength as determined from data derived from load cells and/or other pressure sensing elements.
Embodiments of the invention can be described with reference to the following numbered clauses, with preferred features laid out in the dependent clauses:

### CLAUSES

1. A patient support apparatus, comprising:
   a plurality of load cells positioned on the patient support apparatus,
   a plurality of sensors configured to measure movement of one or more body parts of a patient, and
   a control system including a controller operable to receive a separate signal from each of the plurality of load cells positioned on the patient support apparatus and each of the plurality of sensors, the control system further including a processor and a memory device, the memory device including a set of instructions that, when executed by the processor, causes the control system to determine at least one patient motion and calculate an assessment factor associated with the patient on the patient support apparatus.
2. The patient support apparatus of clause 1, wherein the set of instructions in the memory device is updated in periodic intervals.
3. The patient support apparatus of clause 1, wherein the set of instructions in the memory device is updated in real time.
4. The patient support apparatus of clause 1, wherein the set of instructions in the memory device is provided by an Artificial Intelligence (AI) classifier and wherein an output from the processor is an input to the Artificial Intelligence (AI) classifier.
5. The patient support apparatus of clause 4, wherein the output from the processor is configured to update a first set of instructions in the memory device to a second set of instructions in the memory device.
6. The patient support apparatus of clause 4, wherein the output comprises one or more body parts that are associated with the at least one patient motion.
7. The patient support apparatus of clause 6, wherein the output comprises an amplitude of movement of the one or more body parts associated with the at least one patient motion.
8. The patient support apparatus of clause 6, wherein the output comprises a duration of movement of the one or more body parts associated with the at least one patient motion.
9. The patient support apparatus of clause 6, wherein the output comprises a frequency of movement of the one or more body parts associated with the at least one patient motion.
10. The patient support apparatus of clause 6, wherein the at least one patient motion depends on an amplitude of movement of one or more body parts of the patient.
11. The patient support apparatus of clause 1, wherein the at least one patient motion depends on a duration of movement of one or more body parts of the patient.
12. The patient support apparatus of clause 1, wherein the at least one patient motion depends on a frequency of movement of one or more body parts of the patient.
13. The patient support apparatus of clause 1, wherein the at least one patient motion is identified as an assisted motion or an unassisted motion.
14. The patient support apparatus of clause 13, wherein the at least one patient motion is further identified as a transitory movement or a non-transitory movement.
15. The patient support apparatus of clause 1, wherein the assessment factor is a patient risk factor.
16. The patient support apparatus of clause 15, wherein the patient risk factor is decreased if the at least one patient motion is identified as an assisted motion.
17. The patient support apparatus of clause 15, wherein the patient risk factor is decreased if the at least one patient motion is identified as a transitionary motion.
18. The patient support apparatus of clause 15, wherein the patient risk factor is decreased if the at least one patient motion is identified as an unassisted motion.
19. The patient support apparatus of clause 15, wherein the patient risk factor is decreased if the at least one patient motion is identified as an unassisted motion and a transitionary motion.
20. The patient support apparatus of clause 1, wherein the assessment factor is a patient recovery factor.
21. The patient support apparatus of clause 20, wherein the patient recovery factor is increased if the at least one patient motion is identified as an unassisted motion.
22. The patient support apparatus of clause 20, wherein the patient recovery factor is increased if the at least one patient motion is identified as an unassisted motion and a non-transitionary motion.
23. The patient support apparatus of clause 1, wherein the plurality of sensors includes a plurality of air pressure sensors, each air pressure sensor configured to measure a pressure in an inflatable zone of a mattress positioned on the patient support apparatus.
24. A method of assessing patient recovery, comprising:
   receiving a separate signal from one or more sensors by a control system operable to receive a plurality of signals,
   processing the signal to determine movement of one or more body parts of a patient by a processor in the control system based on a set of instructions stored in a memory device of the control system,
   providing an output about at least one patient motion from the processor, and
   calculating an assessment factor associated with the patient.
25. The method of clause 24, wherein the set of instructions in the memory device is provided by an Artificial Intelligence (AI) classifier, and wherein the output from the processor is an input to the Artificial Intelligence (Al) classifier.
26. The method of clause 24, wherein the set of instructions in the memory device are updated in periodic intervals.
27. The method of clause 24, wherein the set of instructions in the memory device are updated in real time.
28. The method of clause 24, wherein the output from the processor is configured to update a first set of instructions in the memory device to a second set of instructions in the memory device.
29. The method of clause 24, wherein the output comprises one or more body parts that are associated with the at least one patient motion.
30. The method of clause 29, wherein the output comprises an amplitude of movement of the one or more body parts associated with the at least one patient motion.
31. The method of clause 29, wherein the output comprises a duration of movement of the one or more body parts associated with the at least one patient motion.
32. The method of clause 29, wherein the output comprises a frequency of movement of the one or more body parts associated with the at least one patient motion.
33. The method of clause 24, wherein the at least one patient motion depends on an amplitude of movement of one or more body parts of the patient.
34. The method of clause 24, wherein the at least one patient motion depends on a duration of movement of one or more body parts of the patient.
35. The method of clause 24, wherein the at least one patient motion depends on a frequency of movement of one or more body parts of the patient.
36. The method of clause 24, wherein the at least one patient motion is identified as an assisted motion or an unassisted motion.
37. The method of clause 36, wherein the at least one patient motion is further identified as a transitory movement or a non-transitory movement.
38. The method of clause 24, wherein the assessment factor is a patient risk factor.
39. The method of clause 38, wherein the patient risk factor is decreased if the at least one patient motion is identified as an assisted motion.
40. The method of clause 38, wherein the patient risk factor is decreased if the at least one patient motion is identified as a transitionary motion.
41. The method of clause 38, wherein the patient risk factor is decreased if the at least one patient motion is identified as an unassisted motion.
42. The method of clause 38, wherein the patient risk factor is decreased if the at least one patient motion is identified as an unassisted motion and a transitionary motion.
43. The method of clause 24, wherein the assessment factor is a patient recovery factor.
44. The method of clause 43, wherein the patient recovery factor is increased if the at least one patient motion is identified as an unassisted motion.
45. The method of clause 43, wherein the patient recovery factor is increased if the at least one patient motion is identified as an unassisted motion and a non-transitionary motion.
46. The method of clause 24, wherein the one or more sensors includes a plurality of air pressure sensors, each air pressure sensor configured to measure a pressure in an inflatable zone of a mattress positioned on a patient support apparatus.
47. A patient support apparatus, comprising:
   a plurality of sensors configured to measure movement of one or more body parts of a patient on the patient support apparatus, and
   a control system including a controller operable to receive a separate signal from each of the plurality of sensors, the control system further including a processor and a memory device, the memory device including a set of instructions that, when executed by the processor, causes the control system to determine at least one patient motion associated with the patient on the patient support apparatus.
48. The patient support apparatus of clause 47, wherein the set of instructions in the memory device is updated in periodic intervals.
49. The patient support apparatus of clause 47, wherein the set of instructions in the memory device is updated in real time.
50. The patient support apparatus of clause 47, wherein the set of instructions in the memory device is provided by an Artificial Intelligence (AI) classifier and wherein an output from the processor is an input to the Artificial Intelligence (AI) classifier.
51. The patient support apparatus of clause 50, wherein the output from the processor is configured to update a first set of instructions in the memory device to a second set of instructions in the memory device.
52. The patient support apparatus of clause 50, wherein the output comprises one or more body parts that are associated with the at least one patient motion.
53. The patient support apparatus of clause 52, wherein the output comprises an amplitude of movement of the one or more body parts associated with the at least one patient motion.
54. The patient support apparatus of clause 52, wherein the output comprises a duration of movement of the one or more body parts associated with the at least one patient motion.
55. The patient support apparatus of clause 52, wherein the output comprises a frequency of movement of the one or more body parts associated with the at least one patient motion.
56. The patient support apparatus of clause 47, wherein the at least one patient motion depends on an amplitude of movement of one or more body parts of the patient.
57. The patient support apparatus of clause 47, wherein the at least one patient motion depends on a duration of movement of one or more body parts of the patient.
58. The patient support apparatus of clause 47, wherein the at least one patient motion depends on a frequency of movement of one or more body parts of the patient.
59. The patient support apparatus of clause 47, wherein the at least one patient motion is identified as an assisted motion or an unassisted motion.
60. The patient support apparatus of clause 49, wherein the at least one patient motion is further identified as a transitory movement or a non-transitory movement.
61. The patient support apparatus of clause 47, wherein the control system is configured to calculate an assessment factor associated with the patient.
62. The patient support apparatus of clause 61, wherein the assessment factor is a patient risk factor.
63. The patient support apparatus of clause 62, wherein the patient risk factor is decreased if the at least one patient motion is identified as an assisted motion.
64. The patient support apparatus of clause 62, wherein the patient risk factor is decreased if the at least one patient motion is identified as a transitionary motion.
65. The patient support apparatus of clause 62, wherein the patient risk factor is decreased if the at least one patient motion is identified as an unassisted motion.
66. The patient support apparatus of clause 62, wherein the patient risk factor is decreased if the at least one patient motion is identified as an unassisted motion and a transitionary motion.
67. The patient support apparatus of clause 61, wherein the assessment factor is a patient recovery factor.
68. The patient support apparatus of clause 67, wherein the patient recovery factor is increased if the at least one patient motion is identified as an unassisted motion.
69. The patient support apparatus of clause 67, wherein the patient recovery factor is increased if the at least one patient motion is identified as an unassisted motion and a non-transitionary motion.
70. The patient support apparatus of clause 47, wherein the plurality of sensors includes a plurality of load cells.
71. The patient support apparatus of clause 47, wherein the plurality of sensors includes a plurality of air pressure sensors, each air pressure sensor configured to measure a pressure in an inflatable zone of a mattress positioned on the patient support apparatus.
72. The patient support apparatus of clause 50, wherein the output comprises risk associated with pressure related injuries.
73. The patient support apparatus of clause 72, wherein the risk associated with pressure injury is used to automatically adjust the patient support apparatus.
74. A patient support apparatus, comprising:
   a base frame;
   a plurality of casters supporting the base frame;
   an upper frame adjustable relative to the base frame;
   a plurality of movable siderails positioned near the sides of the upper frame;
   a plurality of load cells positioned on the patient support apparatus and configured to sense a patient weight;
   a plurality of sensors configured to measure movement of one or more body parts of a patient;
   a user interface configured to control the patient support apparatus and including a display; and
   a control system including a controller operable to receive a separate signal from each of the plurality of load cells positioned on the patient support apparatus and each of the plurality of sensors, the control system further including a processor and a memory device, the memory device including a set of instructions, wherein the instructions are generated by artificial intelligence or utilize Artificial Intelligence (Al), wherein the instructions, when executed by the processor, cause the control system to determine at least one patient motion and calculate a score associated with the risk of the patient on the patient support apparatus developing a pressure ulcer.
75. The patient support apparatus of clause 74, further comprising a plurality of air bladders, wherein the score is used to adjust pressure in the air bladders.
76. The patient support apparatus of clause 74, wherein the score is used to adjust therapy delivered by patient support apparatus.
77. The patient support apparatus of clause 74, wherein the score is displayed on the user interface.
78. The patient support apparatus of clause 74, wherein the score is sent to a remote computer system or electronic medical record.
79. The patient support apparatus of clause 74, wherein the set of instructions in the memory device is updated in periodic intervals.
80. The patient support apparatus of clause 74, wherein the set of instructions in the memory device is updated in real time.
81. The patient support apparatus of clause 74, wherein an output from the processor is an input to the Artificial Intelligence (AI) classifier.
82. The patient support apparatus of clause 81, wherein the output from the processor is configured to update a first set of instructions in the memory device to a second set of instructions in the memory device.
83. The patient support apparatus of clause 81, wherein the output comprises one or more body parts that are associated with the at least one patient motion.
84. The patient support apparatus of clause 83, wherein the output comprises an amplitude of movement of the one or more body parts associated with the at least one patient motion.
85. The patient support apparatus of clause 83, wherein the output comprises a duration of movement of the one or more body parts associated with the at least one patient motion.
86. The patient support apparatus of clause 83, wherein the output comprises a frequency of movement of the one or more body parts associated with the at least one patient motion.
87. The patient support apparatus of clause 83, wherein the at least one patient motion depends on an amplitude of movement of one or more body parts of the patient.
88. The patient support apparatus of clause 74, wherein the at least one patient motion depends on a duration of movement of one or more body parts of the patient.
89. The patient support apparatus of clause 74, wherein the at least one patient motion depends on a frequency of movement of one or more body parts of the patient.
90. The patient support apparatus of clause 74, wherein the at least one patient motion is identified as an assisted motion or an unassisted motion.
91. The patient support apparatus of clause 90, wherein the at least one patient motion is further identified as a transitory movement or a non-transitory movement.
92. The patient support apparatus of clause 74, wherein the score is a patient risk factor.
93. The patient support apparatus of clause 92, wherein the patient risk factor is decreased if the at least one patient motion is identified as an assisted motion.
94. The patient support apparatus of clause 92, wherein the patient risk factor is decreased if the at least one patient motion is identified as a transitionary motion.
95. The patient support apparatus of clause 92, wherein the patient risk factor is decreased if the at least one patient motion is identified as an unassisted motion.
96. The patient support apparatus of clause 92, wherein the patient risk factor is decreased if the at least one patient motion is identified as an unassisted motion and a transitionary motion.
97. The patient support apparatus of clause 74, wherein the score is a patient recovery factor.
98. The patient support apparatus of clause 97, wherein the patient recovery factor is increased if the at least one patient motion is identified as an unassisted motion.
99. The patient support apparatus of clause 97, wherein the patient recovery factor is increased if the at least one patient motion is identified as an unassisted motion and a non-transitionary motion.

## Claims

1. A patient support apparatus, comprising:
a plurality of load cells positioned on the patient support apparatus,
a plurality of sensors configured to measure movement of one or more body parts of a patient, and
a control system including a controller operable to receive a separate signal from each of the plurality of load cells positioned on the patient support apparatus and each of the plurality of sensors, the control system further including a processor and a memory device, the memory device including a set of instructions that, when executed by the processor, causes the control system to determine at least one patient motion and calculate an assessment factor associated with the patient on the patient support apparatus.

2. The patient support apparatus of claim 1, wherein the set of instructions in the memory device is provided by an Artificial Intelligence (AI) classifier and wherein an output from the processor, comprising one or more body parts that are associated with the at least one patient motion is an input to the Artificial Intelligence (AI) classifier.

3. The patient support apparatus of claim 2, wherein the output comprises one of an amplitude of movement of the one or more body parts associated with the at least one patient motion, a duration of movement of the one or more body parts associated with the at least one patient motion, and a frequency of movement of the one or more body parts associated with the at least one patient motion.

4. The patient support apparatus of claim 2, wherein the output comprises identifying one or more body parts that are associated with the at least one patient motion.

5. 5 The patient support apparatus of any proceeding claim , wherein the at least one patient motion depends on an amplitude of movement of one or more body parts of the patient, a duration of movement of one or more body parts of the patient, or a frequency of movement of one or more body parts of the patient.

6. The patient support apparatus of any proceeding claim , wherein the at least one patient motion is identified as an assisted motion or an unassisted motion.

7. The patient support apparatus of any proceeding claim, wherein the assessment factor is a patient risk factor, wherein the patient risk factor is decreased if the at least one patient motion is identified as an assisted motion, the at least one patient motion is identified as a transitionary motion, if the at least one patient motion is identified as an unassisted motion, or if the at least one patient motion is identified as an unassisted motion and a transitionary motion.

8. The patient support apparatus of any proceeding claim , wherein the assessment factor is a patient recovery factor, wherein the patient recovery factor is increased if the at least one patient motion is identified as an unassisted motion or if the at least one patient motion is identified as an unassisted motion and a non-transitionary motion.

9. A method of assessing patient recovery, comprising:
receiving a signal from one or more sensors by a control system operable to receive a plurality of signals,
processing the signal to determine movement of one or more body parts of a patient by a processor in the control system based on a set of instructions stored in a memory device of the control system,
providing an output about at least one patient motion from the processor, and
calculating, using Artificial Intelligence (AI) an assessment factor associated with the patient.

10. The method of claim 9, wherein the set of instructions in the memory device is updated in periodic intervals.

11. The method of either claim 9 or claim 10, wherein the set of instructions in the memory device is updated in real time.

12. The method of any one of claims 9 to 11, wherein the output from the processor is configured to update a first set of instructions in the memory device to a second set of instructions in the memory device.

13. The method of any one of claims 9 to 12, wherein the output comprises one or more body parts that are associated with the at least one patient motion.

14. The method of any one of claims 9 to 13, wherein the assessment factor is a patient risk factor or patient recovery factor.

15. The method of any one of claims 9 to 14, wherein the one or more sensors includes a plurality of air pressure sensors, each air pressure sensor configured to measure a pressure in an inflatable zone of a mattress positioned on a patient support apparatus.
